**Europäisches Patentamt**

**European Patent Office**

**Office européen des brevets**

(19)

(11) Veröffentlichungsnummer: **0 172 931**
**B1**

(12)

# EUROPÄISCHE PATENTSCHRIFT

(45) Veröffentlichungstag der Patentschrift:
06.04.88

(21) Anmeldenummer: **84110196.7**

(22) Anmeldetag: **28.08.84**

(51) Int. Cl.⁴: **C 07 C  51/573,** C 07 C  57/155

(54) **Verfahren zur Gewinnung von 5-Ring-Dicarbonsäureanhydriden.**

(43) Veröffentlichungstag der Anmeldung:
**05.03.86 Patentblatt 86/10**

(45) Bekanntmachung des Hinweises auf die Patenterteilung:
**06.04.88 Patentblatt 88/14**

(84) Benannte Vertragsstaaten:
**AT BE CH DE FR GB IT LI NL**

(56) Entgegenhaltungen:
**EP-A-0 174 379**
**DE-A-3 321 703**

(73) Patentinhaber: **HÜLS AKTIENGESELLSCHAFT, - RSP Patente / PB 15 - Postfach 13 20, D-4370 Marl 1 (DE)**

(72) Erfinder: **Gude, Fritz, Dr., Wilhelmstrasse 4, D-4690 Herne 2 (DE)**
Erfinder: **von Praun, Dr., Ferdinand, Am Schmöningsberg 8, D-4358 Haltern 6 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Nach der katalytischen Luftoxidation von o-Xylol oder o-Xylol enthaltenden Einsatzprodukten bei etwa 400°C schlägt man das entstandene Phthalsäureanhydrid in der Technik durch Kühlen des Reaktionsgases in einen Rippenrohrkondensator nieder. Aus dem auf diese Weise weitgehend von Phthalsäureanhydrid befreiten Reaktionsgas werden durch eine Wasserwäsche praktisch alle organischen Substanzen herausgelöst, wobei die Anhydride zu Carbonsäuren hydrolysieren. Die erhaltene wäßrige Lösung behandelt man nun i. a. mit Katalysatoren, wie z. B. Thioharnstoff und Schwefeldioxid, um die durch Abbau entstandene Maleinsäure in schwerlösliche Fumarsäure umzulagern. Nach deren Abtrennung wird das Filtrat nach dem Stand der Technik ins Abwasser geleitet. Es enthält nach reiner o-Xyloloxidation noch 16 bis 40 g/l Citraconsäure, 2 bis 14 g/l Phthalsäure, 1 bis 7 g/l Benzoesäure, 2 bis 10 g/l Fumarsäure und kleinere Mengen weiterer Substanzen.

Aus Umweltschutzgründen und weil das Abwasser noch eine Menge wertvoller Substanzen - vor allem Citraconsäure- enthält, ist es sinnvoll, nach technisch einfachen Aufarbeitungsverfahren zu suchen. Immerhin fallen bei der Herstellung von 1 000 t Phthalsäureanhydrid durch Oxidation von o-Xylol etwa 2 bis 4 t Citraconsäure an.

Die Isolierung der Säure durch Abdestillieren des Wassers verbietet sich schon aus Wirtschaftlichen und energetischen Gründen. Wegen der Flüchtigkeit der freien Citraconsäure mit Wasserdampf müßte vorher auch noch mit Alkalien neutralisiert und nach dem Abdampfen wieder angesäuert werden, um das Gemisch der freien Säure gewinnen zu können. Eine andere Methode beschreibt die DE-AS 15 93 246. Dort wird Citraconsäureanhydrid aus der wäßrigen Lösung der Citraconsäure durch azeotrope Destillation des gesamten Wassers mit Hilfe von Benzol, Toluol, Chlorbenzol, Dichlorethan, Trichlorethan und/oder Hexan gewonnen. Die DE-AS 15 93 546 schlägt vor, 80 bis 90 % des Lösungswassers im Vakuum bei einer Sumpftemperatur unterhalb 80°C abzudestillieren, dann oberhalb einer Sumpftemperatur von 80°C gemeinsam mit dem Wasser die Citraconsäure als Anhydrid überzudestillieren und zur Gewinnung des öligen, wasserunlöslichen Citraconsäureanhydrids das Wasser abzuscheiden. Bei den bekannten Verfahren wird also in aufwendiger Weise die Aufarbeitung der wäßrigen Citraconsäurelösung durch Abdestillieren des Wassers durchgeführt. Dagegen sollte die Gewinnung der Citraconsäure und der anderen im Fumarsäureabwasser enthaltenen Säuren durch Extraktion mit geeigneten basischen Mitteln möglich sein. Um das Wasser abtrennen zu können, dürfen das Extraktionsmittel und die Salze aus Extraktionsmittel und Carbonsäuren nicht darin löslich sein. Eine chemische Reaktion der Base mit der Säure soll nicht eintreten.

Nun hat M. Z. Jakuschkin (SU-PS 168 674) über die Extraktion von niedrigen aliphatischen Monocarbonsäuren - wie Ameisen-, Essig-, Propion- und Buttersäure - aus wäßrigen Lösungen mit Trioctylamin berichtet. Anschließend isolierte er das wasserunlösliche Salz und erhitzte es auf etwa 290°C, wobei sich die Säuren abspalteten und weitgehend überdestillierten.

Es stellte sich nun heraus, daß sich auch Dicarbonsäuren, wie z. B. Citraconsäure, Itaconsäure, Phthalsäure usw. mit Tri-n-octylamin aus wäßrigen Lösungen extrahieren lassen. So kann z. B. Fumarsäureabwasser zur Entfernung aller sauren Inhaltsstoffe mit dieser Base ausgeschüttelt werden. Nach Abtrennen der öligen wasserunlöslichen Schicht versetzt man mit einem geeigneten sich bereits bei 105 bis 110°C/15 mbar überraschenderweise Citraconsäureanhydrid und bei ca. 160°C im Vakuum Phthalsäureanhydrid destillativ abtrennen, nachdem als Zwischenlauf noch etwas Benzoesäure angefallen war. Bei Einsatz geeigneter aliphatischer Lösemittel kann aus dem Abwasserextrakt das Citraconsäureanhydrid auch in heteroazeotroper Mischung mit dem Lösemittel und Wasser abgetrieben werden. Nach der Destillation ist aber in beiden Fällen das Amin im Sumpf teilweise zersetzt. Ähnlich instabil sind unter diesen Bedingungen noch viele andere tert. Amine. Überraschenderweise erweisen sich jedoch tert. Amine mit in 2-Stellung verzweigten primären aliphatischen Seitenketten, wie z. B. Tri-(2-ethylhexyl)-amin, Tri-(2-ethylbutyl)-amin, Tri-(2-ethyldecyl)-amin usw. unter den Verfahrensbedingungen als stabil. Alle anderen Reaktionen, also die Extraktion der Säuren aus der wäßrigen Lösung und die Aufarbeitung der 1,2-Di-carbonsäuren durch Destillation als Anhydride, können mit dieser Substanzklasse wie beim Einsatz von Tri-n-octylamin durchgeführt werden. Im Gegensatz zu den stärker basischen, unverzweigten tertiären Aminen ist mit ihnen allerdings keine vollständige Extraktion der sauren Bestandteile des Fumarsäure-Abwassers möglich.

Ein Vorteil der Verwendung der oben aufgeführten verzweigten Amine ist, daß die Spaltung der Salze bei thermischer Belastung ca. 20 bis 30°C früher in ihre Komponenten erfolgt als beim Einsatz der übrigen tert. Amine. Die bei niedrigerer Temperatur eintretende Freisetzung der Dicarbonsäuren schont die Basen und erhöht die Ausbeute an gewinnbaren Anhydriden, wenn die anschließende Destillation bei möglichst niedrigen Temperaturen, also bei einem guten technischen Vakuum, durchgeführt wird.

Eine alternative schonendere Methode zur Aufarbeitung des Salzgemisches ist z. B. folgende: nach Abdestillieren des Wasser/Kohlenwasserstoff-Azeotrops sowie des überschüssigen Schleppmittels wird bei gutem Vakuum wie oben das Citraconsäureanhydrid abdestilliert. Anschließend leitet man bei erhöhter Temperatur unter Vakuum ein indifferentes Gas, wie z. B. Stickstoff, Wasserstoff, Kohlenoxid, Kohlendioxid, Luft usw. in das tert. Amin, wobei die Benzoesäure und das Phthalsäureanhydrid absublimieren. Bei Verwendung von Luft ist der Einsatz von tert. Aminen mit in 2-Stellung verzweigten, aliphatischen Seitengruppen von großem Vorteil, weil sich diese Substanzklasse unerwarteterweise im Gegensatz zu unverzweigten tert. Aminen gegen Luft-Oxidationen als erheblich stabiler erwiesen hat.

Sinngemäß kann man in gleicher Art auch andere 5-Ring-Dicarbonsäureanhydride als die Säuren des Fumarsäureabwassers aus den wäßrigen Lösungen ihrer Dicarbonsäuren, wie z. B. Bernsteinsäure, Itaconsäure, Methylbernsteinsäure usw. als 5-Ring-Dicarbonsäureanhydride gewinnen, allerdings zum Teil mit deutlich geringerer Ausbeute.

Wie sich herausstellte, erfolgt ggf. nach der destillativen Entfernung des Schleppmittels eine Phasentrennung zwischen den 5-Ring-Dicarbonsäureanhydriden und den Aminen oberhalb der Schmelzpunkte der Anhydride. Zur schonenderen Aufarbeitung können nun nach Separierung die Anhydride durch Destillation gereinigt werden, während die Amine unmittelbar oder nach vorheriger Reinigung in den Extraktionsprozeß zurückgeführt werden können.

Die vorliegende Erfindung betrifft daher ein Verfahren zur Gewinnung von 5-Ring-Dicarbonsäureanhydriden mit Ausnahme von Maleinsäureanhydrid aus wäßrigen Lösungen ihrer Dicarbonsäuren, wobei man die wäßrige Lösung mit wasserunlöslichen tert. Aminen im Molverhältnis tert. Amin zu Dicarbonsäure von mindestens 1 : 1 wäscht, das entstandene Salz vom Wasser abtrennt, ein aromatisches Schleppmittel für Wasser zusetzt und, nach Abdestillieren des Schleppmittel/Wasser-Azeotrops, im Vakuum die entstandenen 5-Ring-Dicarbonsäureanhydride von dem tert. Amin abtrennt.

Im Falle der Gewinnung von Citraconsäureanhydrid kann wegen seiner relativ hohen Hydrolysestabilität auch so verfahren werden, daß nach der Extraktion der Citraconsäure mit dem tert. Amin unter Zusatz von aliphatischen Kohlenwasserstoffen, z. B. Dodecan, als Schleppmittel eine Trennung von dem Amin durch Destillation erfolgt. Dabei geht ein ternäres Azeotrop - bestehend aus Citraconsäureanhydrid, Dehydratisierungswasser und Schleppmittel über. Nach Phasentrennung läßt sich das Citraconsäureanhydrid abscheiden.

Will man aus dem Fumarsäureabwasser ausschließlich Citraconsäure abscheiden, so bietet sich eine Wäsche mit den oben aufgeführten, schwachbasischen tert. Aminen mit in 2-Stellung verzweigten primären aliphatischen Seitenketten an. Unter Verzicht auf eine vollständige Gewinnung kann man auf diese Weise die in dem Fumarsäureabwasser vorhandene Citraconsäure fast rein erhalten. Den Rest leitet man mit den übrigen Säuren ins Abwasser.

In einer von D. Rittenberg und L. Ponticorvo in den Proceedings of the National Academy of Sciences (USA) 46 (1960), Seiten 822 bis 824, veröffentlichten Arbeit wird beschrieben, daß viele cyclische aliphatische Anhydride, u. a. auch Citraconsäureanhydrid, bei Behandlung mit katalytischen Mengen tert. Amine unter Kohlendioxidentwicklung reagieren. Diese Reaktion tritt bei dem erfindungsgemäßen Verfahren nur dann störend in Erscheinung, wenn die Aufarbeitung des Aminextraktes mit zu hoher und zu langer thermischer Belastung erfolgt.

Die Zersetzungsreaktion wird ebenfalls stark zurückgedrängt, wenn man anstelle der katalytischen Mengen tert. Amine die zur Erzielung guter Extraktionsergebnisse notwendige Menge von 1 Mol tert. Amin und mehr pro Mol Dicarbonsäure einsetzt.

**Beispiel 1**

1 l eines Abwassers aus der Fumarsäurefabrikation, das 27,9 g (0,21 Mol) Citraconsäure, 1,7 g Phthalsäure, 1,1 g Benzoesäure, 2,3 g Fumarsäure und kleinere Mengen weiterer Substanzen enthielt, wurde zweimal mit je 177 g (0,5 Mol) Tri-n-octylamin 15 Minuten lang durch Ausrühren extrahiert und auf diese Weise nahezu vollständig entsäuert. Der Aminextrakt wurde anschließend bei einem Druck von 200 mbar in 1 500 ml auf 160 bis 165° C erhitztes Dodecan eingetropft und gleichzeitig ein Destillat abgezogen, das sich in der Vorlage in eine obere Dodecan-, in eine mittlere Wasser- und eine untere Citraconsäureanhydridphase auftrennte. Die Ausbeute an Citraconsäureanhydrid betrug 21,1 g entsprechend 87,8 % der Theorie.

Das lösemittelfreie Sumpfprodukt aus der obigen Destillation wurde unter verbessertem Vakuum bei 150 bis 160° C gehalten und mit einem Stickstoffstrom Benzoesäure und schließlich Phthalsäureanhydrid ausgetrieben. Das als Rückstand in der Destillationsblase verbleibende Tri-n- octylamin zersetzte sich teilweise bei dieser Prozedur.

**Beispiel 2**

1 l des Abwassers aus Beispiel 1 wurde zweimal mit je 177 g (0,5 Mol) Tri-(2-ethylhexyl)-amin 15 Minuten lang durch Ausrühren extrahiert. Der resultierende Aminextrakt, der 69 % der Citraconsäure enthielt, wurde anschließend bei einem Druck von 200 mbar in 1 500 ml auf 160 bis 165° c erhitztes Dodecan eingetropft. Hierbei destillierte gleichzeitig ein heteroazeotropes Gemisch ab, das sich in der Vorlage in eine obere Dodecan-, in eine mittlere Wasser- und eine untere Citraconsäureanhydridphase auftrenne. Die Ausbeute an Anhydrid betrug 15,4 g entsprechend 93 % der Theorie.

Aus dem Sumpfprodukt der obigen Destillation wurden unter verbessertem Vakuum bei 150 bis 160°C mit einem Stickstoffstrom Benzoesäure und danach Phthalsäureanhydrid entfernt. Das zurückbleibende Tri-(2-ethylhexyl)-amin war fast frei von Abbauprodukten und ließ sich ohne Reinigung wiederverwerten.

**Beispiel 3**

Ein Aminextrakt, der im wesentlichen 141 g (0,4 Mol) Tri- n-octylamin und 26 g (0,2 Mol) Citraconsäure enthielt, wurde nach Zugabe von 250 ml Xylol zum Sieden (145°C) erhitzt und die bei der Anhydridbildung entstehende Wassermenge abgetrennt. Anschließend wurde das Xylol destillativ entfernt; das Sumpfprodukt trennte sich bei Raumtemperatur in eine obere Amin und eine untere Anhydridphase auf. Letztere lieferte nach Destillation 10,9 g Citraconsäureanhydrid entsprechend 48,6 % der Theorie. Zusätzlich waren noch 1,4 g (6,2 %) Anhydrid in der dunkel gefärbten Aminphase gelöst. Der Aminverlust lag bei 9,2 %.

**Beispiel 4**

Ein Aminextrakt bestehend aus 141 g (0,4 Mol) Tri-(2-ethylhexyl)-amin und 26 g (0,2 Mol) Citraconsäure wurde nach Zugabe von 250 ml Xylol zum Sieden bei einem Druck von ca. 450 mbar und einer Temperatur von 110 bis 115°C erhitzt. Die bei der Anhydridbildung anfallende Wassermenge wurde ausgekreist und anschließend das Lösemittel abdestilliert. Das Sumpfprodukt trennte sich bei Raumtemperatur in eine obere Amin- und eine untere Anhydridphase auf Letztere lieferte nach Destillation 16,3 g Citraconsäureanhydrid entsprechend 72,8 % der Theorie. Die nur schwach gefärbte Aminphase enthielt noch 1,4 g (6,2 %) Anhydrid; sie ließ sich ohne weitere Reinigung wiederverwenden. Der Aminverlust betrug 0,96 %.

**Beispiel 5**

Ein Aminextrakt bestehend aus 54 g (0,15 Mol) Tri-n- octylamin und 10 g (0,08 Mol) Citraconsäure wurde nach Zugabe von 100 ml Octan zum Sieden (128°C) erhitzt. Die bei der Anhydridbildung anfallende Wassermenge wurde als heteroazeotrope Mischung mit Lösemittel und etwas Anhydrid abgetrennt und letzteres in einer Menge von 0,5 g entsprechend 5,8 % der Theorie isoliert. Nach Abkühlung auf Raumtemperatur trennte sich das Sumpfprodukt in zwei Phasen; die obere dunkel gefärbte Aminphase enthielt 0,94 g (10,9 % der Theorie) Citraconsäureanhydrid, während aus der unteren Phase 4,3 g (49,6 % der Theorie) des Anhydrids destillativ gewonnen werden konnten. Der Aminverlust betrug 7,6 %.

**Beispiel 6**

Ein Aminextrakt bestehend aus 141 g (0,4 Mol) Tri-(2-ethylhexyl)-amin und 26 g (0,2 Mol) Citraconsäure wurde nach Zugabe von 250 ml Octan bei 110°C und schwachem Vakuum zum Sieden erhitzt. Die bei der Anhydridbildung anfallende Wassermenge wurde als hetereoazeotrope Mischung mit Lösemittel und etwas Anhydrid abgetrennt und letzteres in einer Menge von 2,0 g entsprechend 8,9 % der Theorie isoliert. Nach Abkühlung auf Raumtemperatur trennte sich das Sumpfprodukt in eine obere aminhaltige Phase, die noch 1,4 g (6,2 % der Theorie) Anhydrid enthielt, sowie in eine untere Phase, aus der 17,7 g (78,8 % der Theorie) Citraconsäureanhydrid destillativ gewonnen werden konnten.

Die octanhaltige Aminphase ließ sich ohne weitere Reinigung wiederverwenden. Der Aminverlust betrug 0,7 %.

**Beispiel 7**

2 l des Abwassers aus Beispiel 1 wurden mit 353 g (1 Mol) Tri-(2-ethylhexyl)-amin 15 Minuten lang durch Ausrühren extrahiert. Mit dem so erhaltenen Extrakt, der 3,5 % Citraconsäure enthielt, wurden ein weiteres Mal 2 l frisches Abwasser extrahiert, wobei der Citraconsäuregehalt im Extrakt auf 6,3 % stieg. Eine analoge dritte Extraktion ergab eine weitere Steigerung auf 10,6 % Citraconsäure im Extrakt. Die Gesamtextraktionsrate nach den drei Verfahrensschritten lag bei 27,9 %. Die Aufarbeitung des Extraktes kann gemäß den vorherigen Beispielen erfolgen. Eine entsprechende Anreicherung an Citraconsäure gelingt nicht bei Verwendung von Tri-n-octylamin als Extraktionsmittel.

**Patentansprüche**

1. Verfahren zur Gewinnung von 5-Ring-Dicarbonsäureanhydriden mit Ausnahme von Maleinsäureanhydrid aus wäßrigen Lösungen ihrer Dicarbonsäuren,
dadurch gekennzeichnet,
daß man die wäßrige Lösung mit wasserunlöslichen tert. Aminen im Molverhältnis tert. Amin zu Dicarbonsäure von mindestens 1 : 1 wäscht, das entstandene Salz vom Wasser abtrennt,

a) ein aromatisches Schleppmittel für Wasser zusetzt und, nach Abdestillieren des Schleppmittel/Wasser-Azeotrops die entstandenen 5-Ring-Dicarbonsäure-anhydride gg. nach vorheriger Phasentrennung destillativ vom Amin abtrennt, oder im Falle der Gewinnung von Citraconsäureanhydrid,

b) eine aliphatischen Kohlenwasserstoff als Schleppmittel zusetzt, ein ternäres azeotropes Gemisch, bestehend aus hydrolysestabilem Anhydrid, Dehydratisierungswasser und Schleppmittel vom Amin abdestilliert und nich Phasentrennung das Anhydrid abtrennt.

2. Verfahren nach Anspruch 1, dadurch gekennzeichnet, daß als Dicarbonsäure Citraconsäure eingesetzt wird.

3. Verfahren nach Anspruch 1, dadurch gekennzeichnet, daß als tert. Amine solche mit in 2-Stellung verzweigten primären aliphatischen Seitenketten verwendet worden.

## Claims

1. Process for recovering 5-ring-dicarboxylic acid anhydrides, with the exception of maleic anhydride, from aqueous solutions of their dicarboxylic acids, characterized in that, the aqueous solution is washed with water-insoluble tert. amines with a molar ratio of tert. amine to dicarboxylic acid of at least 1 : 1, the resulting salt is separated from the water,

a) an aromatic entrainer for water is added and, after the entrainer/water azeotrope is distilled off the resulting 5-ring-dicarboxylic acid anhydride is, if necessary after prior phase separation, separated by distillation from amine, or in the case of recovering citraconic anhydride

b) an aliphatic hydrocarbon is added as entrainer, a ternary azeotropic mixture consisting of an anhydride stable to hydrolysis, water of dehydration and entrainer is distilled from the amine and after phase separation the anhydride is separated.

2. Process according to Claim 1, characterized in that citraconic acid is used as dicarboxylic acid.

3. Process according to Claim 1, characterized in that as tert. amines, those with a branched primary aliphatic side chain in the 2-position are used.

## Revendications

1. Procédé pour la récupération d'anhydrides dicarboxyliques-5-cycliques, à l'exception de l'anhydride maléïque, à partir de solutions aqueuses de leurs acides dicarboxyliques, procédé caractérisé en ce que l'on lave la solution aqueuse avec des amines tertiaires insolubles à l'eau avec un rapport moléculaire amine tert.: acide dicarboxylique d'au moins 1 : 1, sépare le sel qui s'est formé de l'eau:

a) ajoute un agent d'entraînement de l'eau aromatique, et, après élimination par distillation de l'azéotrope agent d'entraînement/eau, sépare l'anhydride dicarboxy- lique-5-cyclique qui s'est formé, éventuellement après séparation de phase préalable, par distillation de l'amine, ou dans le cas de récupération d'anhydride citraconique,

b) ajoute, comme agent d'entraînement, un hydrocarbure aliphatique, élimine de l'amine, par distillation, un mélange azéotrope ternaire constitué de l'anhydride stable à l'hydrolyse, de l'eau de déhydratation et de l'agent d'entraînement, et sépare l'anhydride après séparation de phase.

2. Procédé suivant la revendication 1, caractérisé en ce que, comme acide dicarboxylique, on utilise l'acide citraconique.

3. Procédé suivant la revendication 1, caractérisé en ce que comme amines tertiaires, on utilise celles qui possèdent des chaînes latérales primaires aliphatiques, bifurquées en position 2.